# EUROPEAN PATENT SPECIFICATION

(11) **EP 4 179 539 B1**
(45) Date of publication and mention of the grant of the patent: **01.10.2025**
(21) Application number: 21742062.9
(22) Date of filing: 30.06.2021
(51) Int. Cl.: G16B 50/50, G16B 30/00, H03M 7/30, H03M 7/40

(54) **GENOMIC INFORMATION COMPRESSION BY CONFIGURABLE MACHINE LEARNING-BASED ARITHMETIC CODING**
KOMPRIMIERUNG GENOMISCHER INFORMATIONEN DURCH KONFIGURIERBARE ARITHMETISCHE CODIERUNG AUF BASIS VON MASCHINENLERNEN
COMPRESSION D'INFORMATIONS GÉNOMIQUES PAR CODAGE ARITHMÉTIQUE BASÉ SUR UN APPRENTISSAGE MACHINE CONFIGURABLE

(30) Priority: 10.07.2020 US 202063050193 P
(43) Date of publication of application: 17.05.2023
(73) Proprietor: Koninklijke Philips N.V., 5656 AG Eindhoven (NL)
(72) Inventor: CHANDAK, Shubham, 5656 AE Eindhoven (NL); CHEUNG, Yee, Him, 5656 AE Eindhoven (NL)
(74) Representative: Philips Intellectual Property & Standards
(86) International application number: PCT/EP2021/067960
(87) International publication number: WO 2022/008311

(56) References cited:
- WO-A1-2012/172114
- WO-A1-2018/151788
- WO-A1-2021/156110
- CN-A- 108 306 650
- YANG W ET AL: "Improving Coding Efficiency of MPEG-G Standard Using Context-Based Arithmetic Coding", 2018 IEEE INTERNATIONAL CONFERENCE ON BIOINFORMATICS AND BIOMEDICINE (BIBM), IEEE, 3 December 2018 (2018-12-03), pages 1177 - 1183, XP033507766, DOI: 10.1109/BIBM.2018.8621550
- CHEUNG P Y H (PHILIPS) ET AL: "Response to Call for Evidence: Support for Generic Machine Learning Models for Data Compression", no. m56641, 20 April 2021 (2021-04-20), XP030295150, Retrieved from the Internet <URL:https://dms.mpeg.expert/doc_end_user/documents/134_OnLine/wg11/m56641-v2-M56641SupportforMLModels.zip M56641 Support for ML Models.docx> [retrieved on 20210420]
- CHEUNG P Y H (PHILIPS) ET AL: "Response to Call for Evidence: Improved Quality Value Compression Framework", no. m56642, 20 April 2021 (2021-04-20), XP030295152, Retrieved from the Internet <URL:https://dms.mpeg.expert/doc_end_user/documents/134_OnLine/wg11/m56642-v2-M56642ImprovedQualityValueCompressionFramework.zip M56642 Improved Quality Value Compression Framework.docx> [retrieved on 20210420]

## Description

### TECHNICAL FIELD

Various exemplary embodiments disclosed herein relate generally to a system and method for an extensible framework for context selection, model training and machine learning-based arithmetic coding for MPEG-G.

### BACKGROUND

High throughput sequencing has made it possible to scan genetic material at ever decreasing cost, leading to an ever increasing amount of genetic data, and a need to efficiently compress this data, but preferably also in a manner compatible with envisaged use. Applications occur e.g. in medicine (detection of diseases), and monitoring of the population (e.g. SARS-COV-2 detection), forensics, etc.

Since DNA (deoxyribonucleic acid) and RNA (ribonucleic acid) are built of only 4 different nucleobases (cytosine [C], guanine [G], adenine [A] and thymine [T] for DNA respectively adenine, cytosine, guanine, and uracil [U] for RNA), one could naively think encoding would be easy. However, the genetic information comes in ever new different forms. For example, raw data may come from different sequencing techniques, such as second-generation versus long-read sequencing, which results in different lengths of reads, but also having different base call certainty, which is added to the base sequence or multiple sequences as quality information like quality scores, which must also be encoded. Furthermore, in downstream analysis of the DNA, information may be generated about properties of the DNA, such as differences in comparison with a reference sequence. One can then annotate, for example, that one or more bases are missing compared to the reference. A single-nucleotide variant may be known to lead to a disease or some other genetically determined property, and this can be annotated in a manner so that the information is easily found by another user of the encoded data. Epigenetics, which studies external modifications to DNA sequences, again produces a rich amount of additional data, like e.g. methylation, chromosome contact matrix that reveals the spatial organization of chromatin in a cell, etc. All of these applications will in the future create rich data sets which need powerful encoding techniques.

MPEG-G is a recent initiative of the moving pictures expert group to come to a universal representation of genetic information based on a thorough debate of the various needs of the users. Context-adaptive binary arithmetic coding (CABAC) is currently used as the entropy coding mechanism for the compression of descriptors in MPEG-G. However, the current standard allows only the previous symbols as the context in most cases.

WO2018/151788 teaches a method for compressing genomics data which splits the data into items (e.g. the position of matching against a reference chromosome, the type of mismatch such as a deletion of a number of nucleotide bases), and selects separately optimized entropy encoders for all those items.

CN108306650 teaches a method of encoding the qualities of call of nucleotide bases of a sequence, by recognizing that they occur in runs of successive qualities of the same value, and they separately entropy encode the run length and the quality value of that run.

Yang W. et al.: Improving Coding Efficiency of MPEG-G Standard Using Context-Based Arithmetic Coding, 2018 IEEE INTERNATIONAL CONFERENCE ON BIOINFORMATICS AND BIOMEDICINE is another method for entropy coding of runs of quality values.

### SUMMARY

The invention is defined in the appended claims.

A summary of various exemplary embodiments is presented below. Some simplifications and omissions may be made in the following summary, which is intended to highlight and introduce some aspects of the various exemplary embodiments, but not to limit the scope of the invention. Detailed descriptions of an exemplary embodiment adequate to allow those of ordinary skill in the art to make and use the inventive concepts will follow in later sections.

Various embodiments relate to a method for decoding MPEG-G encoded data, including: receiving MPEG-G encoded data; extracting encoding parameters from the encoded data; selecting an arithmetic coding type based upon the extracted encoding parameters; selecting a predictor type based upon the extracted encoding parameters; selecting a context based upon the extracted encoding parameters; and decoding the encoded data using the selected predictor and the selected contexts. The technical element encoding parameters comprises such parameters which are needed for a receiving decoder to determine its decoding process, and in particular may comprise parameters controlling the selection or configuration of various alternative decoding algorithms. Encoded data may specifically mean arithmetically encoded data. Arithmetic encoding maps a sequence of symbols (e.g. A, T, C, G) to an interval in the range [0.0-1.0], based on the probabilities of occurrence of those symbols. It is a property of probability-based encoding that one can optimize the needed amount of bits, by giving less likely to occur symbols more bits in the encoded bit string, and more likely symbols less bit, i.e. one uses the probability estimates to guide this principle. Probabilities can be changed over time, i.e. during the running decoding process. Context adaptive arithmetic encoding is able to further optimize the probabilities based on the identification of different situations, i.e. different contexts (when using the word context we mean it in the sense of arithmetic encoding, i.e. arithmetic encoding context). Conventionally, the context was formed by the results of the previously decoded symbols. For example, if a set of low quality scores was found for the previous bases, it may be reasonable to assume that the reading is still not very certain for the current read base, i.e. that in the genomic information it will also have a low quality score. Ergo, one could set the probabilities for low score values high, where high score values indicate high certainty about the current base. According to the inventors it is however possible to define many more different contexts which can also take into account other data, such as decoded values of other quantities than the quality scores like genomic position of the chromosome currently being decoded.

Arithmetic encoding type specifies to the decoder, as communicated in the encoding parameters present in a communicated encoded MPEG-G data signal, which type of various possible manners of arithmetic encoding of the data was used by the encoder which generated the encoded data. Various embodiments are described, wherein the arithmetic encoding type is one of binary coding and a multi-symbol coding. In multi-symbol coding one defines an alphabet of symbols which one would encounter in the uncoded signal. For example, for DNA nucleobases these symbols could contain symbols for a definite read base, e.g. T for Thymine, or a symbol for an uncertain read base, and for quality scores one can define a set of quantized values for the scores. In binary arithmetic coding, those N alphabet symbols are as a pre-processing step transformed into binary numbers by an elected binarization scheme, e.g. N symbols can be represented by an increasing set of binary ones followed by a zero, e.g. T=0, C=10, G= 110, A=1110.

The inventors have also found that together with or separate from the selection and communication of better contexts, one may also optimize by selecting one of several different predictor types, e.g. through a modelType parameter that indicates whether the predictor being used is one of a count-based type or machine learning model type, such as a specific neural network (topology and/or optimized weights being communicated) to predict the fixed or constantly varying probabilities of the various symbols, based on whichever contexts being used. These contents can be used as input to the neural network, or to select one of several alternative neural networks, or influence a property of the neural network. Other machine learning techniques may alternatively be used to predict the probabilities, i.e. form the predictor model or type. So the predictor type can indicate a main type (neural network versus conventional count-based probability re-estimation), and a sub-type with more details (specifically for neural networks).

Various embodiments are described, wherein when the predictor type identifies a machine learning model, the encoding parameters further include a definition of the machine learning model. By communicating parameters which define the machine learning model (e.g. parameters specifying the topology like connections with hidden layers, the fixed or initial weights for the connections, etc.), the encoder can select a very good model, and communicate it to the decoder, which can then configurate this model prior to starting the decoding of the incoming encoded data. Parameters in the encoded data signal may also repetitively reset or reconfigure the model.

Various embodiments are described, wherein the extracted encoding parameters includes training mode data. The training mode refers to how the model will dynamically adjust itself to the changing data (i.e. train itself to the varying probabilities of the original uncoded data, as used in the encoded data), or stay relatively fixed (e.g. a neural network with weights which were optimized once by the encoder for the entire data set, and communicated to the decoder to be used during the entire decoding). E.g., the neural network may be trained in an outer processing loop over the first 2000 symbols, and then substitute new optimal weights prior to decoding the 2001^{st} encoded bit.

Various embodiments are described, wherein the training mode data includes an initialization type that includes one of a static training mode, semi-adaptive training mode, and adaptive training mode. A typical example of static mode may be where there is a standard pre-defined model, potentially selectable from a set of standard models, used by both encoder and decoder, and the selected model may be communicated to the decoder by e.g. a model number which specifies the selected model. An example of a semi-adaptive model may be where a model is trained using the data being compressed. In this case the weights are optimized for this specific data set.

Various embodiments are described, wherein the training mode data includes one of a training algorithm definition, training algorithm parameters, training frequency, and training epochs. The training frequency is how frequently the model (at the decoding side) should update, e.g. after every 1000 symbols. A training epoch is a concept of machine learning, which specifies the number of times the entire training data set is processed by the machine learning algorithm to update the model.

Various embodiments are described, wherein the extracted encoding parameters includes context data.

Various embodiments are described, wherein the context data includes one of a coding order, number of additional contexts used, context type, and range.

Various embodiments are described, wherein the context data includes a range flag.

Various embodiments are described, wherein the context data includes one of a context descriptor, context output variable, context internal variable, context computed variable, and context computation function.

Further various embodiments relate to a method for encoding MPEG-G encoded data, including: receiving encoding parameters to be used to encode data; selecting an arithmetic encoding type based upon the received encoding parameters; selecting a predictor type based upon the received encoding parameters; selecting a training mode based upon the received encoding parameters; selecting a context based upon the received encoding parameters; training the encoder based upon the received encoding parameters; and encoding the data using the trained encoder.

Various embodiments are described, wherein the arithmetic encoding type is one of binary coding and a multi-symbol coding.

Various embodiments are described, wherein the predictor type is one of a count-based type or machine learning model type.

Various embodiments are described, wherein when the predictor type identifies a machine learning model, the encoding parameters further include a definition of the machine learning model.

Various embodiments are described, wherein the extracted encoding parameters includes training mode data.

Various embodiments are described, wherein the training mode data includes an initialization type that includes one of a static training mode, semi-adaptive training mode, and adaptive training mode.

Various embodiments are described, wherein the training mode data includes one of a training algorithm definition, training algorithm parameters, training frequency, and training epochs.

Various embodiments are described, wherein the extracted encoding parameters includes context data.

Various embodiments are described, wherein the context data includes one of a coding order, number of additional contexts used, context type, and range.

Various embodiments are described, wherein the context data includes a range flag.

Various embodiments are described, wherein the context data includes one of a context descriptor, context output variable, context internal variable, context computed variable, and context computation function.

Further various embodiments relate to a system for decoding MPEG-G encoded data, including: a memory; a processor coupled to the memory, wherein the processor is further configured to: receive MPEG-G encoded data; extract encoding parameters from the encoded data; select an arithmetic encoding type based upon the extracted encoding parameters; select a predictor type based upon the extracted encoding parameters; select a context based upon the extracted encoding parameters; and decode the encoded data using the selected predictor and the selected contexts.

Various embodiments are described, wherein the arithmetic encoding type is one of binary coding and a multi-symbol coding.

Various embodiments are described, wherein the predictor type is one of a count-based type or machine learning model type.

Various embodiments are described, wherein when the predictor type identifies a machine learning model, the encoding parameters further include a definition of the machine learning model.

Various embodiments are described, wherein the extracted encoding parameters includes training mode data.

Various embodiments are described, wherein the training mode data includes an initialization type that includes one of a static training mode, semi-adaptive training mode, and adaptive training mode.

Various embodiments are described, wherein the training mode data includes one of a training algorithm definition, training algorithm parameters, training frequency, and training epochs.

Various embodiments are described, wherein the extracted encoding parameters includes context data.

Various embodiments are described, wherein the context data includes one of a coding order, number of additional contexts used, context type, and range.

Various embodiments are described, wherein the context data includes a range flag.

Various embodiments are described, wherein the context data includes one of a context descriptor, context output variable, context internal variable, context computed variable, and context computation function.

Further various embodiments relate to a system for encoding MPEG-G encoded data, including: a memory; a processor coupled to the memory, wherein the processor is further configured to: receive encoding parameters to be used to encode data; select an arithmetic encoding type based upon the received encoding parameters; select a predictor type based upon the received encoding parameters; select a training mode based upon the received encoding parameters; select a context based upon the received encoding parameters; train the encoder based upon the received encoding parameters; and encode the data using the trained encoder.

Various embodiments are described, wherein the arithmetic encoding type is one of binary coding and a multi-symbol coding.

Various embodiments are described, wherein the predictor type is one of a count-based type or machine learning model type.

Various embodiments are described, wherein when the predictor type identifies a machine learning model, the encoding parameters further include a definition of the machine learning model.

Various embodiments are described, wherein the extracted encoding parameters includes training mode data.

Various embodiments are described, wherein the training mode data includes an initialization type that includes one of a static training mode, semi-adaptive training mode, and adaptive training mode.

Various embodiments are described, wherein the training mode data includes one of a training algorithm definition, training algorithm parameters, training frequency, and training epochs.

Various embodiments are described, wherein the extracted encoding parameters includes context data.

Various embodiments are described, wherein the context data includes one of a coding order, number of additional contexts used, context type, and range.

Various embodiments are described, wherein the context data includes a range flag.

Various embodiments are described, wherein the context data includes one of a context descriptor, context output variable, context internal variable, context computed variable, and context computation function.

### BRIEF DESCRIPTION OF THE DRAWINGS

In order to better understand various exemplary embodiments, reference is made to the accompanying drawings, wherein:
FIG. 1 illustrates a block diagram of CABAC;
FIG. 2 illustrates a block diagram of a manner of selection of a predictor model, encoding mode, training mode and predictive contexts, and their associated parameters;
FIG. 3 illustrates a method for encoding data using the modified MPEG-G standard;
FIG. 4 illustrates a method for decoding data using the modified MPEG-G standard;
FIG. 5 illustrates an exemplary hardware diagram for the encoding/decoding system; and
Fig. 6 shows a scheme of sub-circuits of an embodiment using as probability model a neural network.

To facilitate understanding, identical reference numerals have been used to designate elements having substantially the same or similar structure and/or substantially the same or similar function.

### DETAILED DESCRIPTION

The description and drawings illustrate the principles of the invention. It will thus be appreciated that those skilled in the art will be able to devise various arrangements that, although not explicitly described or shown herein, embody the principles of the invention and are included within its scope. Furthermore, all examples recited herein are principally intended expressly to be for pedagogical purposes to aid the reader in understanding the principles of the invention and the concepts contributed by the inventor(s) to furthering the art and are to be construed as being without limitation to such specifically recited examples and conditions. Additionally, the term, "or," as used herein, refers to a non-exclusive or *(i.e.,* and/or), unless otherwise indicated (e.g., "or else" or "or in the alternative"). Also, the various embodiments described herein are not necessarily mutually exclusive, as some embodiments can be combined with one or more other embodiments to form new embodiments.

Context-adaptive binary arithmetic coding (CABAC) is currently used as the entropy coding mechanism for the compression of descriptors in MPEG-G. However, the current standard is severely limited in terms of the choice of contexts, allowing only the previous symbols as the context in most cases. This does not allow the use of other contexts such as different descriptors which may provide a boost in the compression ratios. Furthermore, the current framework lacks support for more powerful predictors such as neural networks and different training modes. A framework is described herein for incorporating these additional functionalities into the MPEG-G standard, enabling greater flexibility and improved compression. The embodiments described herein are not limited to the MPEG-G standard, but may be applied to other compressed file formats as well.

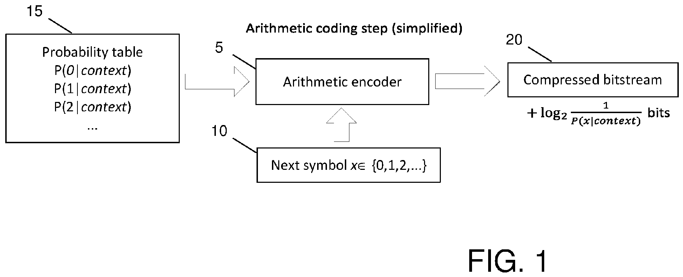

The MPEG-G standard for genomic data compresses the genomic data in terms of different descriptors. The compression engine is context-adaptive binary arithmetic coding (CABAC), which is based on arithmetic coding. Arithmetic coding is a standard approach for data compression which performs optimal compression under a (possibly adaptive) probabilistic model for the data. The better the model predicts the data, the better is the compression. The model might incorporate various contexts that have statistical correlation with the data to be compressed, and the current standard allows the use of the previous symbols as a context for the probability model needed in the arithmetic coding. FIG. 1 illustrates a block diagram of CABAC. The arithmetic encoder 5 takes the next symbol 10 as an input *(i.e.,* x ∈ 0,1,2,...}). The arithmetic encoder 5 uses a probability table that provides the probability of a specific symbol occurring in a specific context. Using these inputs, the encoder 5 then produces the compressed bitstream 20. For some specific descriptors like mmtype, the standard also allows the use of additional contexts such as reference base, but in general there is a lack of support for using other descriptors as context as well as for other additional contexts. This is despite the fact that compression may be improved by inclusion of such additional contexts, such as where the position in the read is used as a context for quality value compression. Similarly, for nanopore data, the sequence bases may be used as a context for improved quality value compression. It is to be expected that there exists many more such correlations across descriptors that may be exploited for improving the compression.

Furthermore, the current standard only allows adaptive arithmetic coding setup while there exist several modes for arithmetic coding as described below. One possible mode is static modeling that uses a fixed model accessible to encoder and decoder. This static model is suitable when a lot of similar data is available for training. Another possible mode is semi-adaptive modeling where the model is learned from data to be compressed and the model parameters are stored as part of compressed file. This semi-adaptive model is suitable when similar data for model training is not available. Finally, there is adaptive modeling where the encoder/decoder start with same random model and the model is updated adaptively based on data seen up to the current time. As a result, there is no need to store the model as the model updates are symmetric. This adaptive mode is suitable when similar data is not available and/or when using a simple predictor *(e.g.,* a count based predictor). Therefore, depending on the availability of prior training data, different modelling techniques may be more appropriate in different situations. Note that the adaptive updates to the model may also be made in the static and semi-adaptive settings.

Another limitation of the current standard is the lack of support for more complex probability predictors such as neural networks or other machine learning models. Currently only the count-based framework is supported where the probability of the next symbol is computed based on empirical probabilities from counts. These counts are updated at every step based on the context and the next symbol. However, such a count-based approach suffers from two major limitations.

First, the count-based approach is unable to exploit the similarities and dependencies across contexts. For example, the counts for the contexts (A, A, A, A) and (A, A, A, C) are treated as being independent even though it may be expected that there might be some similarities. Similarly, if the previous quality is used as a context, the values of 39 or 40 are treated independently, without utilizing their closeness. Second, the count-based approach does not work well when the context set is very large (or uncountable) as compared to the data size. This is because the array of counts becomes very sparse leading to insufficient data and poor probability modelling. This limits the use of powerful contexts that can lead to much better prediction and compression.

Both of these issues may be overcome using a neural network/machine learning based approach which provides a much more natural prediction framework. Further, the neural network/machine learning based approach is able to work with different types of contexts such as numerical, categorical, and ordinal. In some cases, this improved compression may be worth the increased computational complexity, especially in cases when specialized hardware or parallel computation is available. Note that the neural network can be trained using the cross-entropy loss which directly corresponds to the compressed size.

To summarize the advantages of the two approaches: the count-based approach is computationally cheap, and it is easy to train the adaptive model. On the other hand, the count-based approach treats each context value independently (which may not be the case and could provide valuable insights) and suffers when there are insufficient counts for various symbols and contexts. The neural network/machine learning approach can capture complex interdependencies across context values, and it works well with large/uncountable contact sets. On the other hand, the neural network/machine learning based approach is computationally expensive and is difficult to train in adaptive modelling.

Finally, there is a lack of support for multi-symbol arithmetic coding in the current standard which may usually provide much better compression and needs much fewer parameters as compared to the binary CABAC entropy coder. The CABAC encoder does have advantages in terms of computation but providing support for multi-symbol arithmetic coding can lead to a better tradeoff between compression ratio and speed.

Embodiments of modifications to the MPEG-G standard are proposed in order to accommodate multiple contexts based on different descriptors that may be used for: arithmetic coding; neural network or machine learning based predictive modelling; support for static, semi-adaptive and adaptive modelling; and multi-symbol arithmetic coding. Overall, this provides a highly extensible framework capable of capturing the correlations between descriptors for improved compression. The static mode also allows the ability to develop a trained model from a collection of datasets and then use it for achieving improved compression.

For simplicity, multi-symbol arithmetic coding will be used in the description, but the binary arithmetic coding can be done in a similar manner. All referenced MPEG-G clauses belong to MPEG-G part 2 (DIS ISO/IEC 23092-2 2^{nd} Edition Coding of Genomic Information).

A first modification is to add an arithmetic coding type that indicates whether the arithmetic coding is binary or multi-symbol. Typically multi-symbol corresponds to encoding a byte at a time, but this can be modified in certain cases. Currently the MPEG-G standard decoder configuration includes only a single mode (CABAC). An additional mode for multi-symbol arithmetic coding is indicated by **encodingMode** = 1. Otherwise when **encodingMode** = 0 CABAC coding is indicated.

Another modification is to add a predictor type that indicates whether the predictor is count-based, neural network, or machine learning based. An additional flag **modelType** is added to the MPEG-G decoder configuration. A value of 0 denotes count-based model while a value of 1 denotes neural-network based model. Note that neural networks as a general category encompassing various architectures and models may encompass several other machine learning frameworks such as logistic regression and SVM. The framework may be extended even further by including additional (non-neural network) machine learning predictors such as decision trees and random forests. Each of these different approaches may have associated **modelType** values that indicate the type of predictor used.

When the **modelType** is 1, *i.e.,* a neural network-based model, the model architecture is also specified as part of the decoder configuration. The model architecture may be stored using JavaScript Object Notation (JSON) using the gen_info datatype from in the MPEG-G standard that allows arbitrary data to be stored and compressed with 7zip. As an example, the Keras function model.to_json() generates a JSON string representing the model architecture. Note that the output size of the neural network should be equal to the number of symbols in the arithmetic coding, because it will be fed to the arithmetic encoder. The input size depends on the context being used. Similar to the neural network based model, other machine learning models may be specified as part of the decoder configuration.

Another modification is to add a training mode that indicates whether the training mode is static, semi-adaptive, or adaptive. This allows for a training mode to be selected.

The training mode can be specified by adding additional flags **initializationType** and **adaptiveLearning** to the decoder configuration. The possible values and the respective description are provided below.

When **initialization Type** = 0, static initialization is indicated. In this case, a standard model available to both the encoder and the decoder is used as the initial model for compression. An additional variable **modelURI** (model uniform resource identifier) is used to gain access to the model parameters (weights), which are usually part of a standard model repository. This may also refer to a randomly initialized model with a known seed. Note that the model architecture is already specified (in e.g., JSON format) as discussed previously.

When **initialization Type** = 1, semi-adaptive initialization is indicated. In this case, a model is stored as part of the compressed file in the variable **savedModel.** The model may be in the Hierarchical Data Format version 5 (HDF5) format for neural networks (as used in Keras for instance). For the count-based framework, the model just consists of the counts for each (context, symbol) pair. The **savedModel** variable is of gen_info type which is compressed with 7-zip to potentially reduce the model size.

To control whether adaptive learning is used during the compression/decompression process, a flag **adaptiveLearning** is used. When set to 1 (true) and **modelType** is 1 (neural network) additional following variables are used to describe the training procedure and frequency: **trainingAlgorithm** selects the algorithm for training *(e.g.,* Adam, stochastic gradient decent (SGD), Adagrad, *etc.);* **trainingAlgorithmParameters** is a set of parameters for the training algorithm in JSON format, in particular the learning rate; **trainingFrequency** is the frequency of a model update step *(e.g.,* after every symbol, after every 1000 symbols, etc.), and at each training step, the previous **"trainingFrequency"** symbols (e.g., the previous 1000 symbols when **trainingFrequency** = 1000) are used as the training data, allowing for efficient updates; and **trainingEpochs** tells how many epochs of training are performed at each model update step. Note that the learning rate should be kept low when the initial model is already trained. In such cases, the adaptive learning should be used for the finetuning of the model.

When **modelType** is 0 indicating a count-based model, the update is performed at every step and the count corresponding to the (context, symbol) pair is incremented by 1. Note that the training is performed independently in each access unit to enable fast selective access.

Currently the only contexts allowed are the previous symbols and the number of these used for the decoding is decided by the **coding_order** variable in the MPEG-G standard which may be 0, 1 or 2. coding_order signals the number of previously decoded symbols internally maintained as state variables and is used to decode the next symbol. In the special case of the variables mmtype and rftt, special dependencies are defined in the MPEG-G standard, however this is not very systematic and these dependencies are still treated as previous symbols which limits the coding order and is semantically incorrect.

A method to incorporate a large number of contexts by introducing new variables in the MPEG-G standard may include the following variables: **coding_order, num_additional_contexts, context_type,** and **range.** The variable **coding_order** has the same meaning as before. The variable **coding_order** may have a value greater than 2 since the neural network-based predictors works quite well with larger contexts. The variable **num**_**additional_contexts** indicates the number of additional contexts used.

The variable **context_type** indicates the type of context and an additional value is added for each additional context. The type of the context may include the following possible categories: descriptor, **output_variable, internal_variable,** and **computed_variable.** The variable **descriptor** indicates that the context is the value of another descriptor (e.g., pos or rcomp). In this case the particular **descriptorID** and subsequenceID is also be specified. The variable **output_variable** indicates that the context is the value of one of the variables in the decoded MPEG-G record, *e.g.,* sequence, quality _values, *etc.* The name of the **output_variable** is specified. The variable **internal_variable** indicates that the context is an internal variable computed during the decoding process (e.g., mismatchOffsets). The name of the internal variable is specified. Note that only the internal variables defined in the standard text are recognized. The variable **computed_variable** is a variable that may be computed from the internal variables but is not itself specified in the standard. In this case, a function that computes this variable is included as **contextComputationFunction** (the executable of this function may be run on a standardized virtual machine to allow interoperability across computing platforms). To prevent malicious code, this function may contain a **digital signature** from a trusted authority. This may be useful to implement complex contexts such as the "average quality score of all previously decoded bases mapping to the current genome position".

The variable **range** indicates a range for each additional context, whenever applicable. This is applicable when the variable is an array and only a subset of values are to be used for the decoding. Along with the start and end positions, the variable **range** uses a **rangeFlag** to denote whether the range is described with respect to the start of the array or with respect to the current position in the array. At the boundary positions of the array, default values (as specified by a **defaultContext** variable) are used if the range exceeds the limits. For example, if the reference sequence of the read sequence is used as a context for compression of quality values, then the range can be specified with respect to the current position - a range of [-3,3] means that we are using a context of size 7 centered at the current position.

Note that the dependency graph for the different variables should not contain any cycles, *i.e.,* the dependency graph should be a directed acyclic graph (DAG). As an example of a valid dependency graph, variable 1 is encoded without any dependencies, variable 2 is encoded dependent on variable 1 as a context, variable 3 is encoded dependent on variables 1 and 2, and variable 4 is encoded dependent on variable 2.

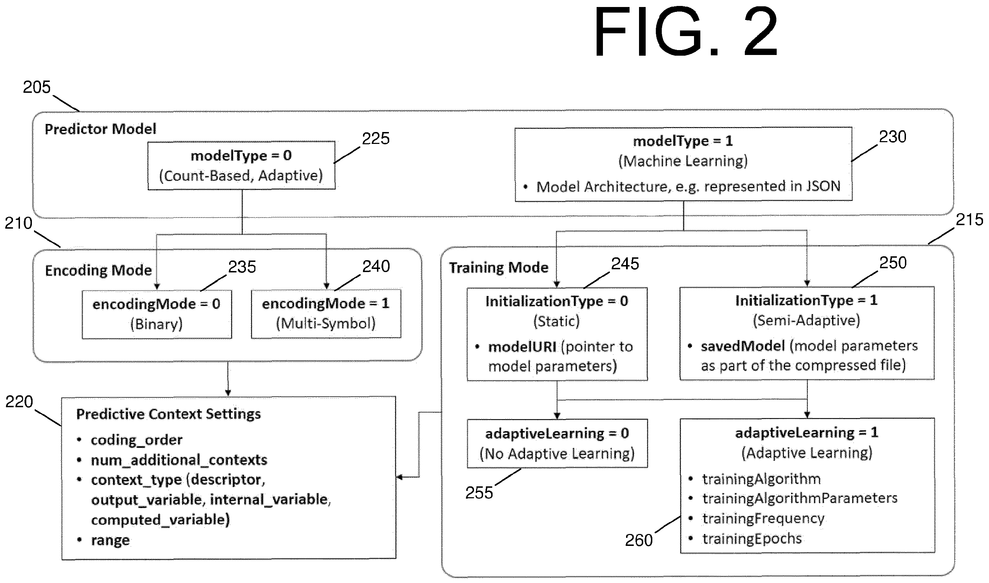

The modification to the MPEG-G standard may be used to improve the compression of various descriptors by selecting contexts that are good predictors for the descriptor. If the computational resources are available, the neural network-based prediction can be used to build a better predictor and also handle large context sets more efficiently. Depending on the availability of similar data for training, the static or the semi-adaptive training procedures can be used. On top of this, adaptive training can be added to further finetune the model, with this being especially useful for count-based models. FIG. 2 illustrates a block diagram of the selection of predictor model, encoding mode, training mode and predictive contexts, and their associated parameters. Note that the purpose of this figure is to illustrate the roles of the key parameters, and the blocks do not necessarily need to be in the exact same order. The block diagram illustrates a predictor model 205, an encoding mode 210, a training mode 215, and predictive context settings 220. In this example in the predictor model 205, when the **modelType** = 0 (i.e., count-based, adaptive) 225, the encoding mode 210 may be entered. In the encoding mode 210, when **encodingMode** = 0 235, the encoding is binary. In the encoding mode 210, when **encodingMode** = 1 240, the encoding is multi-symbol. The encoding mode 210 may then store various predictive context settings 220. The predictive context settings 220 may include coding_order, num_additional_contexts, context_type (descriptor, output_variable, internal_variable, computed_variable), and/or range.

Further, when the **modelType** = 1 230 (i.e., machine learning), the training mode 215 may be entered. In this case, the machine learning model architecture may be specified using for example a JSON representation. In the training mode 215 when **InitializationType** = 0 245, a static initialization is indicated and the **modelURI** points to the model parameters. In the training mode 215 when **InitializationType** = 1 250, a semi-adaptive initialization is indicated and the **savedModel** includes the model parameters as part of the compressed file. Next, when **adaptiveLearning** = 0 255, no adaptive learning is used in the training of the model. When **adaptiveLearning** = 1 260, adaptive learning is used in training the model and the following paraments may be specified: trainingAlgorithm; trainingAlgorithmParameters; trainingFrequency; and trainingEpochs. The training mode 215 may then store needed parameters in the predictive context settings 220.

As described in another U.S. Patent Application No. 62/971,293, filed February 7, 2020, entitled "Improved quality value compression framework in aligned sequencing data based on novel contexts" (which is hereby incorporated for all purposes as if included herein), the quality value compression may be improved by incorporating contexts such as position in read, nearby bases in the read, nearby bases in the reference, the presence and type of error at the base, the average quality value at the genomic coordinate, and other contexts obtained from the alignment information. That patent application also discusses in detail a methodology of selecting the context, and the pros and cons of using count-based prediction as opposed to neural network-based prediction. The results in that disclosure are based on multi-symbol arithmetic coding which is much simpler in terms of parameter optimization as compared to CABAC, while being computationally more expensive.

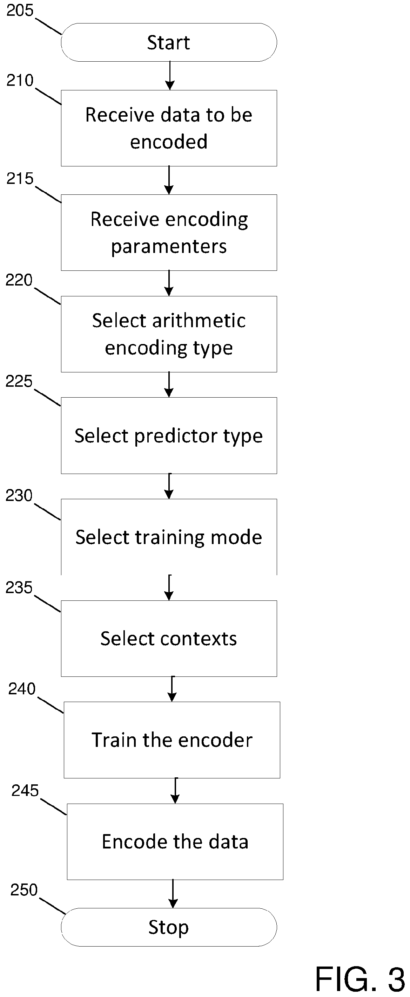

FIG. 3 illustrates a method for encoding data using the modified MPEG-G standard. This is an exemplary method of a versatile encoder. In some embodiments some of the steps may be default. E.g. the selection of the arithmetic encoding type may instead of selecting between various options, use a default selection, like e.g. binary arithmetic coding. Also, the training mode may not always involve a complicated selection, e.g. in the case of a static training it may be prefixed, at least partially. However, some indicator values regarding the training mode may be set according to a universal definition. The encoding method 200 begins at 205, and then the encoding method 200 receives the data to be encoded 210. In this application such data would be various genomic data, related metadata, quality value, *etc.* Next, the encoding parameters may be received 215. The encoding parameters may be selected by a user and provided to the encoding method 200, may be in a configuration file, or may be determined based upon analyzing the type of data to be encoded and/or the computing resources available (e.g., the arithmetic coding type **encodingMode** may be selected based upon the format of the data to be encoded or the **modelType** may be selected based upon the amount of data available for training and the processing resources available). Next, the encoding method 200 selects the arithmetic encoding type 220. This will be based upon the received encoding parameters and may include binary or multi-symbol arithmetic encoding as indicated by the variable **encodingMode.**

The encoding method 200 then selects the predictor type 225 based upon the variable **modelType.** As described above this may indicate CABAC, a neural network based predictor, or some other type of machine learning or other type of predictor. Next, the encoding method 200 selects the training mode 230 based upon the variable **initialization Type.** Also, the variable **adaptiveLearning** indicates whether adaptive learning will be used during the encoding. The method 200 then selects the training mode 230. The training mode is defined by the various training parameters discussed above. Next, the method 200 selects the contexts 235 based upon the various variables defined above.

The method 200 next trains the encoder 240. This training will depend upon the predictor type, *i.e.,* count-based or neural network-based. Further, the various training parameters will define how the training proceeds as well as the training method used. The trained encoder is then used to encode the data 245. If an adaptive predictor is used, the predictor is updated as the encoding proceeds. Further, various encoding parameters as defined above are appended to the encoded data. The method 200 then stops at 250.

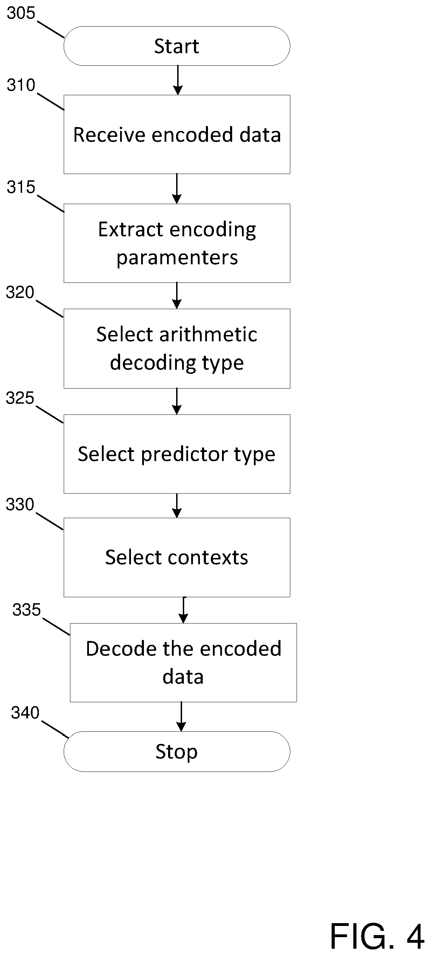

FIG. 4 illustrates a method for decoding data using the modified MPEG-G standard. This is an exemplary decoding. Note that similar to the encoding, some of the steps can be default. E.g. the arithmetic decoding type may be fixed to multilevel (or binary), and only the prediction type and context information are actually communicated by the encoder and preconfigured by the decoder. In such a case the encoding parameters will typically prescribe the prediction type and contexts, but not the arithmetic decoding type. The decoding method 300 begins at 305, and then the decoding method 300 receives the data to be decoded 310. The encoded data may include various genomic data, related metadata, quality value, *etc.* Next, the encoding parameters may be extracted 315 from the encoded data. The decoding method 300 selects the arithmetic encoding type 320. This will be based upon the extracted encoding parameters and may include binary or multi-symbol arithmetic encoding as indicated by the variable **encodingMode.**

The decoding method 300 then selects the predictor type 325 based upon the extracted variable **modelType.** As described above this may indicate count-based predictor, a neural network-based predictor, or some other type of machine learning or other type of predictor. If a neural network or machine learning based predictor is used, the definition of these models are also extracted from the encoding parameters. Next, the method 300 selects the contexts 330 based upon the various variables defined above.

The decoder is then used to decode the data 335 based upon the various encoding parameters and predictor model. If an adaptive predictor is used the predictor is updated as the decoding proceeds. The method 300 then stops at 340.

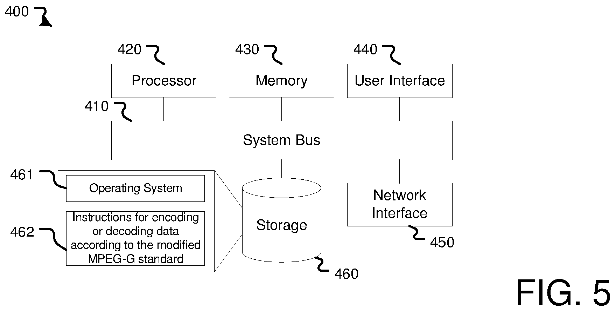

FIG. 5 illustrates an exemplary hardware diagram 400 for the encoding/decoding system. As shown, the device 400 includes a processor 420, memory 430, user interface 440, network interface 450, and storage 460 interconnected via one or more system buses 410. It will be understood that FIG. 5 constitutes, in some respects, an abstraction and that the actual organization of the components of the device 400 may be more complex than illustrated.

The processor 420 may be any hardware device capable of executing instructions stored in memory 430 or storage 460 or otherwise processing data. As such, the processor may include a microprocessor, a graphics processing unit (GPU), field programmable gate array (FPGA), application-specific integrated circuit (ASIC), any processor capable of parallel computing, or other similar devices. The processor may also be a special processor that implements machine learning models.

The memory 430 may include various memories such as, for example L1, L2, or L3 cache or system memory. As such, the memory 430 may include static random-access memory (SRAM), dynamic RAM (DRAM), flash memory, read only memory (ROM), or other similar memory devices.

The user interface 440 may include one or more devices for enabling communication with a user and may present information to users. For example, the user interface 440 may include a display, a touch interface, a mouse, and/or a keyboard for receiving user commands. In some embodiments, the user interface 440 may include a command line interface or graphical user interface that may be presented to a remote terminal via the network interface 450.

The network interface 450 may include one or more devices for enabling communication with other hardware devices. For example, the network interface 450 may include a network interface card (NIC) configured to communicate according to the Ethernet protocol or other communications protocols, including wireless protocols. Additionally, the network interface 450 may implement a TCP/IP stack for communication according to the TCP/IP protocols. Various alternative or additional hardware or configurations for the network interface 450 will be apparent.

The storage 460 may include one or more machine-readable storage media such as read-only memory (ROM), random-access memory (RAM), magnetic disk storage media, optical storage media, flash-memory devices, or similar storage media. In various embodiments, the storage 460 may store instructions for execution by the processor 420 or data upon which the processor 420 may operate. For example, the storage 460 may store a base operating system 461 for controlling various basic operations of the hardware 400. The storage 462 may store instructions for implementing the encoding or decoding data according to the modified MPEG-G standard.

It will be apparent that various information described as stored in the storage 460 may be additionally or alternatively stored in the memory 430. In this respect, the memory 430 may also be considered to constitute a "storage device" and the storage 460 may be considered a "memory." Various other arrangements will be apparent. Further, the memory 430 and storage 460 may both be considered to be "non-transitory machine-readable media." As used herein, the term "non-transitory" will be understood to exclude transitory signals but to include all forms of storage, including both volatile and non-volatile memories.

While the system 400 is shown as including one of each described component, the various components may be duplicated in various embodiments. For example, the processor 420 may include multiple microprocessors that are configured to independently execute the methods described herein or are configured to perform steps or subroutines of the methods described herein such that the multiple processors cooperate to achieve the functionality described herein. Such plurality of processors may be of the same or different types. Further, where the device 400 is implemented in a cloud computing system, the various hardware components may belong to separate physical systems. For example, the processor 420 may include a first processor in a first server and a second processor in a second server.

The encoding/decoding method and system described herein provides a technological improvement over the current MPEG-G standard. The method and system described herein includes the ability to add different predictor models, to allow for different types of arithmetic encoding, and provide the ability to include additional contexts into the training of a predictive model for the encoding/decoding of the genetic data. These and other additional features described herein allow for increased compression of the data taking advantage of other additional information in the data. This allows for reduced storage of the genetic data which has great benefits in the storing of more complete genomes for further analysis. Also, the additional flexibility allows for encoding decisions to be made based upon the available computing and storage resources available to balance between increased compression and the additional computing resources required to achieve increased compression.

Any combination of specific software running on a processor to implement the embodiments of the invention, constitute a specific dedicated machine.

As used herein, the term "non-transitory machine-readable storage medium" will be understood to exclude a transitory propagation signal but to include all forms of volatile and non-volatile memory.

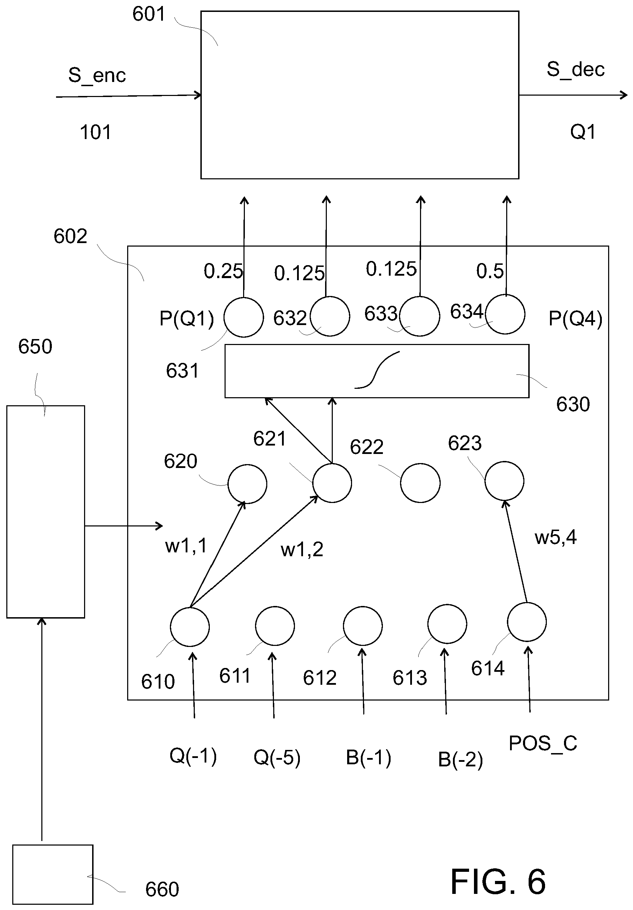

Fig. 6 shows, to illustrate the generic concept of machine learning based adaptable probability models, an example of a context adaptive arithmetic decoder, which uses a neural network as predictor for the probabilities of the symbols in an alphabet of four possible values for a quality value (Q1-Q4). In general, the symbols in the alphabet correspond to the various quantized quality levels, for example, Q1 may be the lowest quality and Q4 may be the highest. Arithmetic decoding circuit 601 again needs to know the current probabilities of those 4 possible output symbols (P(Q1)-P(Q4)), to be able to decode the encoded data S_enc into decoded data S_dec. So, using the principles of arithmetic decoding, it knows from the position in the normalized interval [0.0-1.0], and the corresponding binary representation of the fraction, that the current set of input bits encode a current quality level of, say, Q1. If working on counts, the decoder would typically update the probabilities of the model for the next symbol decoding (e.g. since Q1 was decoded, the lower quality scores Q1 and Q2 may be more likely for the next decoding). The probabilities of the output symbols are inferred by a neural network circuit 602. As explained, various topologies may be used, and various ways of updating, depending on what is most beneficial for encoding and decoding the data. For elucidation in this example the context consists of several categories of input, supplied to the input nodes 610 to 614, after suitable conversion to an input representation, e.g. in the normalized interval. This can use very general contexts. E.g., instead of merely the previous two decoded values of the quantity presently being decoded, the quality scores track, the previous score Q(-1) and the score of 5 positions before Q(-5) may be part of the contexts as inputs to the neural network. There may in some embodiments be a further circuit -not shown- which configures which quantities to send to the input nodes, but since this is a neural network one could immediately input a large set of input quantities, as the neural network can learn that some inputs are unimportant for the prediction by optimizing weights which are (near) zero. One also sees that some input nodes get totally different context quantities, e.g. input nodes 612 and 613 may get the determined nucleobase at previous decoded symbol position B(-1) and the position before B(-2). In this manner the network could learn if a certain sequencing technology has difficulties with accurately determining e.g. a run of N successive Thymine bases, which will show up in the raw quality data, and the statistics for optimal coding (both at encoding and decoding side). Shown is also an example of an extraneous parameter determining the context, namely the position POS_C on a chromosome of which the current set of bases is decoded. The skilled person understands how the same framework can be used for different contexts.

A neural network configuring circuit 650 can periodically set the neural network, so that if needed the neural network can optimize for different probabilistic behaviors of the data set (e.g. the lower part of a chromosome may be better encoded with a differently optimized neural network than the upper part). Depending on the configuration, this circuit may perform different tasks in corresponding sub-units. E.g., it may in parallel run a training phase of exactly the same neural network topology on a set of recent contexts (e.g. for the last 1000 decoded nucleobases and their quality scores in particular). It may then at a time before decoding the present symbol, replace all weights with the optimal values. The neural network configuring circuit 650 typically has access to an encoding parameter data parser 660. In case of a static neural network good for the entire sequence of bases, this parser may read weights from the encoded data signal, and via the neural network configuring circuit 650 load them once in the neural network circuit 602 before the start of decoding. For a neural network probability model, or other machine learning model, that is continuously updating i.e. re-optimizing, the parser may in a similar manner set starting weights for the probability calculation by the neural network circuit 602 for decoding the first few encoded symbols.

Shown in this network topology is one hidden layer (nodes 620-623). It weighs the values of the input nodes by respective weights w1,1 etc. and sums the result. In this manner, by using one or potentially many hidden layers, the network can learn various interdependencies, which can lead to a very good quality probability model for predicting the next symbol. The output nodes will typically follow after an activation function 630, and will represent the probabilities. E.g. output node 631 represents the probability that the current quality would be the first quality (e.g. the worst quality score), and e.g. it may be 0.25. This example merely shows by example one of several variants one can similarly design according to technical principles herein presented. Note also that arithmetic encoding works as a lossless encoding on pure data, i.e. binary or non-binary alphabet symbols, so it can be used both on raw data, and data which has already been predicted by an initial prediction algorithm (i.e. one can run the arithmetic encoder and decoder on both the model parameters of the initial prediction, and/or the residuals between the prediction and the actual raw data).

## Claims

1. A method implemented on a processor for decoding MPEG-G encoded data of genomic information, comprising:
receiving MPEG-G encoded data;
extracting encoding parameters from the encoded data;
selecting a predictor type from the extracted encoding parameters, which specifies the method to obtain probabilities of symbols which were used for arithmetically encoding the data, wherein the prediction type is one of count-based type and machine learning type;
selecting arithmetic coding contexts based upon the extracted encoding parameters; and
decoding the encoded data using the selected predictor and the selected arithmetic coding contexts.

2. The method of claim 1, wherein an arithmetic encoding type is one of binary coding and a multi-symbol coding.

3. The method of claim 1, wherein the predictor type is a neural network.

4. The method of claim 1, wherein when the predictor type identifies a machine learning model, the encoding parameters further include a definition of the machine learning model.

5. The method of claim 1, wherein the extracted encoding parameters includes training mode data, which specifies how the model for predicting probabilities of symbols which are arithmetically encoded varies over time in the decoding.

6. The method of claim 5, wherein the training mode data includes an initialization type that includes one of a static training mode, semi-adaptive training mode, and adaptive training mode.

7. The method of claim 5, wherein the training mode data includes one of a training algorithm definition, training algorithm parameters, training frequency, and training epochs.

8. The method of claim 1, wherein the extracted encoding parameters includes context data.

9. The method of claim 8, wherein the context data includes one of a coding order, number of additional contexts used, context type, and range.

10. The method of claim 8, wherein the context data includes a range flag.

11. The method of claim 8, wherein the context data includes one of a context descriptor, context output variable, context internal variable, context computed variable, and context computation function.

12. A method implemented on a processor
for encoding MPEG-G encoded data of genomic information, comprising
receiving encoding parameters to be used to encode data, where encoding parameters specify how uncoded genomic information is to be encoded;
selecting a predictor type specifying the method to obtain probabilities of symbols which are used for arithmetically encoding the data based upon the received encoding parameters, the predictor type being one of a machine learning prediction and count-based prediction;
selecting arithmetic encoding contexts based upon the received encoding parameters;
training the encoder based upon the received encoding parameters; and
encoding the data using the trained encoder, wherein the encoded data comprises encoding parameters, which specify the method to obtain probabilities of symbols which were used for arithmetically encoding the data.

13. The method of claim 12, wherein an arithmetic encoding type is one of binary coding and a multi-symbol coding.

14. The method of claim 12, wherein when the predictor type identifies a machine learning model, the encoding parameters further include a definition of the machine learning model.

15. The method of claim 12, wherein the extracted encoding parameters includes training mode data.

16. The method of claim 15, wherein the training mode data includes an initialization type that includes one of a static training mode, semi-adaptive training mode, and adaptive training mode.

17. The method of claim 15, wherein the training mode data includes one of a training algorithm definition, training algorithm parameters, training frequency, and training epochs.

18. The method of claim 12, wherein the extracted encoding parameters includes context data.

19. The method of claim 18, wherein the context data includes one of a coding order, number of additional contexts used, context type, and range.

20. The method of claim 18, wherein the context data includes a range flag.

21. The method of claim 18, wherein the context data includes one of a context descriptor, context output variable, context internal variable, context computed variable, and context computation function.

22. A system for decoding MPEG-G encoded data of genomic information, comprising:
a memory;
a processor coupled to the memory, wherein the processor is further configured to:
receive MPEG-G encoded data;
extract encoding parameters from the encoded data;
select a predictor type based upon the extracted encoding parameters which specifies the method to obtain probabilities of symbols which were used for arithmetically encoding the data, wherein the prediction type is one of count-based type and machine learning type;
select arithmetic encoding contexts based upon the extracted encoding parameters; and
decode the encoded data using the selected predictor type and the selected arithmetic encoding contexts.

23. The system of claim 22, wherein an arithmetic encoding type is one of binary coding and a multi-symbol coding.

24. The system of claim 22, wherein when the predictor type identifies a machine learning model, the encoding parameters further include a definition of the machine learning model.

25. The system of claim 22, wherein the extracted encoding parameters includes training mode data.

26. The system of claim 25, wherein the training mode data includes an initialization type that includes one of a static training mode, semi-adaptive training mode, and adaptive training mode.

27. The system of claim 25, wherein the training mode data includes one of a training algorithm definition, training algorithm parameters, training frequency, and training epochs.

28. The system of claim 22, wherein the extracted encoding parameters includes context data.

29. The system of claim 28, wherein the context data includes one of a coding order, number of additional contexts used, context type, and range.

30. The system of claim 28, wherein the context data includes a range flag.

31. The system of claim 28, wherein the context data includes one of a context descriptor, context output variable, context internal variable, context computed variable, and context computation function.

32. A system for encoding MPEG-G encoded data of genomic information, comprising:
a memory;
a processor coupled to the memory, wherein the processor is further configured to:
receive encoding parameters to be used to encode data, where encoding parameters specify how uncoded genomic information is to be encoded;
select a predictor type specifying the method to obtain probabilities of symbols which are used for arithmetically encoding the data based upon the received encoding parameters, wherein the prediction type is one of count-based type and machine learning type;
select arithmetic encoding contexts based upon the received encoding parameters;
train the encoder based upon the received encoding parameters; and
encode the data using the trained encoder, wherein the encoded data comprises encoding parameters, which specify the method to obtain probabilities of symbols which were used for arithmetically encoding the data..

33. The system of claim 32, wherein an arithmetic encoding type is one of binary coding and a multi-symbol coding.

34. The system of claim 32, wherein when the predictor type identifies a machine learning model, the encoding parameters further include a definition of the machine learning model.

35. The system of claim 32, wherein the extracted encoding parameters includes training mode data.

36. The system of claim 35, wherein the training mode data includes an initialization type that includes one of a static training mode, semi-adaptive training mode, and adaptive training mode.

37. The system of claim 35, wherein the training mode data includes one of a training algorithm definition, training algorithm parameters, training frequency, and training epochs.

38. The system of claim 32, wherein the extracted encoding parameters includes context data.

39. The system of claim 38, wherein the context data includes one of a coding order, number of additional contexts used, context type, and range.

40. The system of claim 38, wherein the context data includes a range flag.

41. The system of claim 38, wherein the context data includes one of a context descriptor, context output variable, context internal variable, context computed variable, and context computation function.

## Patentansprüche

1. Verfahren, implementiert auf einem Prozessor, zum Decodieren von MPEG-G-encodierten Daten von genomischen Informationen, umfassend:
Empfangen von MPEG-G encodierten Daten;
Extrahieren von Encodierungsparametern aus den encodierten Daten;
Auswählen eines Prädiktortyps aus den extrahierten Encodierungsparametern, der das Verfahren spezifiziert, um Wahrscheinlichkeiten von Symbolen zu erhalten, die zum arithmetischen Encodieren der Daten verwendet wurden, wobei der Prädiktortyp einer aus einem zählungsbasierten Typ oder einem Maschinenlerntyp ist;
Auswählen von arithmetischen Codierungskontexten basierend auf den extrahierten Encodierungsparametern; und
Decodieren der encodierten Daten unter Verwendung des ausgewählten Prädiktors und der ausgewählten arithmetischen Codierungskontexte.

2. Verfahren nach Anspruch 1, wobei ein arithmetischer Encodierungstyp eine binäre Codierung oder eine Multi-Symbol-Codierung ist.

3. Verfahren nach Anspruch 1, wobei der Prädiktortyp ein neuronales Netzwerk ist.

4. Verfahren nach Anspruch 1, wobei, wenn der Prädiktortyp ein Maschinenlernmodell identifiziert, die Encodierungsparameter weiter eine Definition des Maschinenlernmodells einschließen.

5. Verfahren nach Anspruch 1, wobei die extrahierten Encodierungsparameter Trainingsmodusdaten einschließen, die spezifizieren, wie das Modell zur Vorhersage von Wahrscheinlichkeiten von Symbolen, die arithmetisch encodiert sind, bei der Decodierung über die Zeit variiert.

6. Verfahren nach Anspruch 5, wobei die Trainingsmodusdaten einen Initialisierungstyp einschließen, der einen statischen Trainingsmodus, einen semi-adaptiven Trainingsmodus oder einen adaptiven Trainingsmodus einschließt.

7. Verfahren nach Anspruch 5, wobei die Trainingsmodusdaten eines aus einer Trainingsalgorithmusdefinition, Trainingsalgorithmusparametern, Trainingsfrequenz und Trainingsepochen einschließen.

8. Verfahren nach Anspruch 1, wobei die extrahierten Encodierungsparameter Kontextdaten einschließen.

9. Verfahren nach Anspruch 8, wobei die Kontextdaten eines aus Codierungsreihenfolge, Anzahl der zusätzlich verwendeten Kontexte, Kontexttyp und Bereich einschließen.

10. Verfahren nach Anspruch 8, wobei die Kontextdaten ein Bereichsflag einschließen.

11. Verfahren nach Anspruch 8, wobei die Kontextdaten eines aus einem Kontextdeskriptor, einer Kontextausgabevariable, einer kontextinternen Variable, einer kontextberechneten Variable und einer Kontextberechnungsfunktion einschließen.

12. Verfahren, implementiert auf einem Prozessor, zum Encodieren von MPEG-G encodierten Daten von genomischen Informationen, umfassend:
Empfangen von Encodierungsparametern, die zum Encodieren von Daten verwendet werden sollen, wobei die Encodierungsparameter spezifizieren, wie uncodierte genomische Informationen encodiert werden sollen;
Auswählen eines Prädiktortyps, der das Verfahren zum Erhalten von Wahrscheinlichkeiten von Symbolen spezifiziert, die zum arithmetischen Encodieren der Daten basierend auf den empfangenen Encodierungsparametern verwendet werden, wobei der Prädiktortyp eines aus einer Maschinenlernvorhersage und einer zählungsbasierten Vorhersage ist;
Auswählen von arithmetischen Encodierungskontexten basierend auf den empfangenen Encodierungsparametern;
Trainieren des Encoders basierend auf den empfangenen Encodierungsparametern; und
Encodieren der Daten unter Verwendung des trainierten Encoders, wobei die encodierten Daten Encodierungsparameter umfassen, die das Verfahren zum Erhalten von Wahrscheinlichkeiten von Symbolen spezifizieren, die zum arithmetischen Encodieren der Daten verwendet wurden.

13. Verfahren nach Anspruch 12, wobei ein arithmetischer Encodierungstyp eine binäre Codierung oder eine Multi-Symbol-Codierung ist.

14. Verfahren nach Anspruch 12, wobei, wenn der Prädiktortyp ein Maschinenlernmodell identifiziert, die Encodierungsparameter weiter eine Definition des Maschinenlernmodells einschließen.

15. Verfahren nach Anspruch 12, wobei die extrahierten Encodierungsparameter Trainingsmodusdaten einschließen.

16. Verfahren nach Anspruch 15, wobei die Trainingsmodusdaten einen Initialisierungstyp einschließen, der einen statischen Trainingsmodus, einen semi-adaptiven Trainingsmodus oder einen adaptiven Trainingsmodus einschließt.

17. Verfahren nach Anspruch 15, wobei die Trainingsmodusdaten eines aus einer Trainingsalgorithmusdefinition, Trainingsalgorithmusparametern, Trainingsfrequenz und Trainingsepochen einschließen.

18. Verfahren nach Anspruch 12, wobei die extrahierten Encodierungsparameter Kontextdaten einschließen.

19. Verfahren nach Anspruch 18, wobei die Kontextdaten eines aus Codierungsreihenfolge, Anzahl der zusätzlich verwendeten Kontexte, Kontexttyp und Bereich einschließen.

20. Verfahren nach Anspruch 18, wobei die Kontextdaten ein Bereichsflag einschließen.

21. Verfahren nach Anspruch 18, wobei die Kontextdaten eines aus einem Kontextdeskriptor, einer Kontextausgabevariable, einer kontextinternen Variable, einer kontextberechneten Variable und einer Kontextberechnungsfunktion einschließen.

22. System zum Decodieren von MPEG-G-encodierten Daten von genomischen Informationen, umfassend:
einen Speicher;
einen mit dem Speicher gekoppelten Prozessor, wobei der Prozessor weiter konfiguriert ist zum:
Empfangen von MPEG-G-encodierten Daten;
Extrahieren von Encodierungsparametern aus den encodierten Daten;
Auswählen eines Prädiktortyps basierend auf den extrahierten Encodierungsparametern, der das Verfahren spezifiziert, um Wahrscheinlichkeiten von Symbolen zu erhalten, die zum arithmetischen Encodieren der Daten verwendet wurden, wobei der Prädiktortyp einer aus einem zählungsbasieren Typ und einem Maschinenlerntyp ist;
Auswählen von arithmetischen Encodierungskontexten basierend auf den extrahierten Encodierungsparametern; und
Decodieren der encodierten Daten unter Verwendung des ausgewählten Prädiktortyps und der ausgewählten arithmetischen Encodierungskontexte.

23. System nach Anspruch 22, wobei ein arithmetischer Encodierungstyp eine binäre Codierung oder eine Multi-Symbol-Codierung ist.

24. System nach Anspruch 22, wobei, wenn der Prädiktortyp ein Maschinenlernmodell identifiziert, die Encodierungsparameter weiter eine Definition des Maschinenlernmodells einschließen.

25. System nach Anspruch 22, wobei die extrahierten Encodierungsparameter Trainingsmodusdaten einschließen.

26. System nach Anspruch 25, wobei die Trainingsmodusdaten einen Initialisierungstyp einschließen, der einen statischen Trainingsmodus, einen semi-adaptiven Trainingsmodus oder einen adaptiven Trainingsmodus einschließt.

27. System nach Anspruch 25, wobei die Trainingsmodusdaten eines von einer Trainingsalgorithmusdefinition, Trainingsalgorithmusparametern, Trainingsfrequenz und Trainingsepochen einschließen.

28. System nach Anspruch 22, wobei die extrahierten Encodierungsparameter Kontextdaten einschließen.

29. System nach Anspruch 28, wobei die Kontextdaten eines aus Codierungsreihenfolge, Anzahl der zusätzlich verwendeten Kontexte, Kontexttyp und Bereich einschließen.

30. System nach Anspruch 28, wobei die Kontextdaten ein Bereichsflag einschließen.

31. System nach Anspruch 28, wobei die Kontextdaten eines aus einem Kontextdeskriptor, einer Kontextausgabevariable, einer kontextinternen Variable, einer kontextberechneten Variable oder einer Kontextberechnungsfunktion einschließen.

32. System zum Encodieren von MPEG-G-encodierten Daten von genomischen Informationen, umfassend:
einen Speicher;
einen mit dem Speicher gekoppelten Prozessor, wobei der Prozessor weiter konfiguriert ist zum:
Empfangen von Encodierungsparameters, die zum Encodieren von Daten verwendet werden sollen, wobei die Encodierungsparameter spezifizieren, wie uncodierte genomische Informationen zu encodieren sind;
Auswählen eines Prädiktortyps, der das Verfahren spezifiziert, um Wahrscheinlichkeiten von Symbolen zu erhalten, die für das arithmetische Encodieren der Daten basierend auf den empfangenen Encodierungsparametern verwendet werden, wobei der Prädiktortyp eines aus einem zählungsbasierten Typ und einem Maschinenlerntyp ist;
Auswählen von arithmetischen Encodierungskontexten basierend auf empfangenen Encodierungsparametern;
Trainieren des Encoders basierend auf den empfangenen Encodierungsparametern; und
Encodieren der Daten unter Verwendung des trainierten Encoders, wobei die encodierten Daten Encodierungsparameter umfassen, die das Verfahren zum Erhalten von Wahrscheinlichkeiten von Symbolen spezifizieren, die zum arithmetischen Encodieren der Daten verwendet wurden.

33. System nach Anspruch 32, wobei ein arithmetischer Encodierungstyp eine binäre Codierung oder eine Multi-Symbol-Codierung ist.

34. System nach Anspruch 32, wobei, wenn der Prädiktortyp ein Maschinenlernmodell identifiziert, die Encodierungsparameter weiter eine Definition des Maschinenlernmodells einschließen.

35. System nach Anspruch 32, wobei die extrahierten Encodierungsparameter Trainingsmodusdaten einschließen.

36. System nach Anspruch 35, wobei die Trainingsmodusdaten einen Initialisierungstyp einschließen, die einen statischen Trainingsmodus, einen semi-adaptiven Trainingsmodus oder einen adaptiven Trainingsmodus einschließt.

37. System nach Anspruch 35, wobei die Trainingsmodusdaten eines aus einer Trainingsalgorithmusdefinition, Trainingsalgorithmusparametern, Trainingsfrequenz und Trainingsepochen einschließen.

38. System nach Anspruch 32, wobei die extrahierten Encodierungsparameter Kontextdaten einschließen.

39. System nach Anspruch 38, wobei die Kontextdaten eines aus Codierungsreihenfolge, Anzahl der zusätzlich verwendeten Kontexte, Kontexttyp und Bereich einschließen.

40. System nach Anspruch 38, wobei die Kontextdaten ein Bereichsflag einschließen.

41. System nach Anspruch 38, wobei die Kontextdaten eines aus einem Kontextdeskriptor, einer Kontextausgabevariable, einer kontextinternen Variable, einer kontextberechneten Variable und einer Kontextberechnungsfunktion einschließen.

## Revendications

1. Procédé mis en œuvre sur un processeur pour décoder des données d'informations génomiques codées MPEG-G, comprenant :
la réception de données codées MPEG-G ;
l'extraction de paramètres de codage des données codées ;
la sélection d'un type de prédicteur à partir des paramètres de codage extraits, qui spécifie le procédé pour obtenir des probabilités de symboles qui ont été utilisés pour coder arithmétiquement les données, dans lequel le type de prédiction est l'un d'un type basé sur le comptage et d'un type d'apprentissage automatique ;
la sélection de contextes de codage arithmétique en fonction des paramètres de codage reçus ;
le décodage des données codées à l'aide du prédicteur sélectionné et des contextes de codage arithmétique sélectionnés.

2. Procédé selon la revendication 1, dans lequel un type de codage arithmétique est l'un d'un codage binaire et d'un codage multi-symboles.

3. Procédé selon la revendication 1, dans lequel le type de prédicteur est un réseau neuronal.

4. Procédé selon la revendication 1, dans lequel lorsque le type de prédicteur identifie un modèle d'apprentissage automatique, les paramètres de codage incluent en outre une définition du modèle d'apprentissage automatique.

5. Procédé selon la revendication 1, dans lequel les paramètres de codage extraits incluent des données de mode de formation, qui spécifient la manière dont le modèle de prédiction des probabilités de symboles qui sont codés arithmétiquement varie au fil du temps lors du décodage.

6. Procédé selon la revendication 5, dans lequel les données de mode de formation incluent un type d'initialisation qui inclut l'un d'un mode de formation statique, d'un mode de formation semi-adaptatif et d'un mode de formation adaptatif.

7. Procédé selon la revendication 5, dans lequel les données de mode de formation incluent un élément parmi une définition d'algorithme de formation, des paramètres d'algorithme de formation, une fréquence de formation et des époques de formation.

8. Procédé selon la revendication 1, dans lequel les paramètres de codage extraits incluent des données de contexte.

9. Procédé selon la revendication 8, dans lequel les données de contexte incluent l'un d'un ordre de codage, d'un nombre de contextes supplémentaires utilisés, d'un type de contexte et d'une plage.

10. Procédé selon la revendication 8, dans lequel les données de contexte incluent un indicateur de plage.

11. Procédé selon la revendication 8, dans lequel les données de contexte incluent l'un d'un descripteur de contexte, d'une variable de sortie de contexte, d'une variable interne de contexte, d'une variable calculée de contexte et d'une fonction de calcul de contexte.

12. Procédé mis en œuvre sur un processeur pour coder des données d'informations génomiques codées MPEG-G, comprenant :
la réception de paramètres de codage à utiliser pour coder des données, les paramètres de codage spécifiant comment les informations génomiques non codées doivent être codées ;
la sélection d'un type de prédicteur spécifiant le procédé pour obtenir des probabilités de symboles qui sont utilisés pour coder arithmétiquement les données sur la base des paramètres de codage reçus, le type de prédicteur étant l'un d'une prédiction d'apprentissage automatique et d'une prédiction basée sur le comptage ;
la sélection des contextes de codage arithmétique en fonction des paramètres de codage reçus ;
la formation du codeur en fonction des paramètres de codage reçus ; et
le codage des données à l'aide du codeur formé, dans lequel les données codées comprennent des paramètres de codage, qui spécifient le procédé pour obtenir des probabilités de symboles qui ont été utilisés pour le codage arithmétique des données.

13. Procédé selon la revendication 12, dans lequel un type de codage arithmétique est l'un d'un codage binaire et d'un codage multi-symboles.

14. Procédé selon la revendication 12, dans lequel lorsque le type de prédicteur identifie un modèle d'apprentissage automatique, les paramètres de codage incluent en outre une définition du modèle d'apprentissage automatique.

15. Procédé selon la revendication 12, dans lequel les paramètres de codage extraits incluent des données de mode de formation.

16. Procédé selon la revendication 15, dans lequel les données de mode de formation incluent un type d'initialisation qui inclut l'un d'un mode de formation statique, d'un mode de formation semi-adaptatif et d'un mode de formation adaptatif.

17. Procédé selon la revendication 15, dans lequel les données de mode de formation incluent un élément parmi une définition d'algorithme de formation, des paramètres d'algorithme de formation, une fréquence de formation et des époques de formation.

18. Procédé selon la revendication 12, dans lequel les paramètres de codage extraits incluent des données de contexte.

19. Procédé selon la revendication 18, dans lequel les données de contexte incluent l'un d'un ordre de codage, d'un nombre de contextes supplémentaires utilisés, d'un type de contexte et d'une plage.

20. Procédé selon la revendication 18, dans lequel les données de contexte incluent un indicateur de plage.

21. Procédé selon la revendication 18, dans lequel les données de contexte incluent l'un d'un descripteur de contexte, d'une variable de sortie de contexte, d'une variable interne de contexte, d'une variable calculée de contexte et d'une fonction de calcul de contexte.

22. Système de décodage de données d'informations génomiques codées MPEG-G, comprenant :
une mémoire ;
un processeur couplé à la mémoire, dans lequel le processeur est en outre configuré pour :
recevoir des données codées MPEG-G ;
extraire des paramètres de codage des données codées ;
sélectionner un type de prédicteur sur la base des paramètres de codage extraits, qui spécifie le procédé pour obtenir des probabilités de symboles qui ont été utilisés pour coder arithmétiquement les données, dans lequel le type de prédiction est l'un d'un type basé sur le comptage et d'un type d'apprentissage automatique ;
sélectionner des contextes de codage arithmétique en fonction des paramètres de codage extraits ; et
décoder les données codées à l'aide du type de prédicteur sélectionné et des contextes de codage arithmétique sélectionnés.

23. Système selon la revendication 22, dans lequel un type de codage arithmétique est l'un d'un codage binaire et d'un codage multi-symboles.

24. Système selon la revendication 22, dans lequel lorsque le type de prédicteur identifie un modèle d'apprentissage automatique, les paramètres de codage incluent en outre une définition du modèle d'apprentissage automatique.

25. Système selon la revendication 22, dans lequel les paramètres de codage extraits incluent des données de mode de formation.

26. Système selon la revendication 25, dans lequel les données de mode de formation incluent un type d'initialisation qui inclut l'un d'un mode de formation statique, d'un mode de formation semi-adaptatif et d'un mode de formation adaptatif.

27. Système selon la revendication 25, dans lequel les données de mode de formation incluent un élément parmi une définition d'algorithme de formation, des paramètres d'algorithme de formation, une fréquence de formation et des époques de formation.

28. Système selon la revendication 22, dans lequel les paramètres de codage extraits incluent des données de contexte.

29. Système selon la revendication 28, dans lequel les données de contexte incluent l'un d'un ordre de codage, d'un nombre de contextes supplémentaires utilisés, d'un type de contexte et d'une plage.

30. Système selon la revendication 28, dans lequel les données de contexte incluent un indicateur de plage.

31. Système selon la revendication 28, dans lequel les données de contexte incluent l'un d'un descripteur de contexte, d'une variable de sortie de contexte, d'une variable interne de contexte, d'une variable calculée de contexte et d'une fonction de calcul de contexte.

32. Système de codage de données d'informations génomiques codées MPEG-G, comprenant :
une mémoire ;
un processeur couplé à la mémoire, dans lequel le processeur est en outre configuré pour :
recevoir des paramètres de codage à utiliser pour coder des données, dans lequel les paramètres de codage spécifient comment les informations génomiques non codées doivent être codées ;
sélectionner un type de prédicteur spécifiant le procédé pour obtenir des probabilités de symboles qui sont utilisées pour coder arithmétiquement les données en fonction des paramètres de codage reçus, dans lequel le type de prédiction est l'un d'un type basé sur le comptage et d'un type d'apprentissage automatique ;
sélectionner des contextes de codage arithmétique en fonction des paramètres de codage reçus ;
former le codeur en fonction des paramètres de codage reçus ; et
coder les données à l'aide du codeur formé, dans lequel les données codées comprennent des paramètres de codage, qui spécifient le procédé pour obtenir des probabilités de symboles qui ont été utilisés pour le codage arithmétique des données.

33. Système selon la revendication 32, dans lequel un type de codage arithmétique est l'un parmi un codage binaire et un codage multi-symboles.

34. Système selon la revendication 32, dans lequel lorsque le type de prédicteur identifie un modèle d'apprentissage automatique, les paramètres de codage incluent en outre une définition du modèle d'apprentissage automatique.

35. Système selon la revendication 32, dans lequel les paramètres de codage extraits incluent des données de mode de formation.

36. Système selon la revendication 35, dans lequel les données de mode de formation incluent un type d'initialisation qui inclut l'un d'un mode de formation statique, d'un mode de formation semi-adaptatif et d'un mode de formation adaptatif.

37. Système selon la revendication 35, dans lequel les données de mode de formation incluent un élément parmi une définition d'algorithme de formation, des paramètres d'algorithme de formation, une fréquence de formation et des époques de formation.

38. Système selon la revendication 32, dans lequel les paramètres de codage extraits incluent des données de contexte.

39. Système selon la revendication 38, dans lequel les données de contexte incluent l'un d'un ordre de codage, d'un nombre de contextes supplémentaires utilisés, d'un type de contexte et d'une plage.

40. Système selon la revendication 38, dans lequel les données de contexte incluent un indicateur de plage.

41. Système selon la revendication 38, dans lequel les données de contexte incluent l'un d'un descripteur de contexte, d'une variable de sortie de contexte, d'une variable interne de contexte, d'une variable calculée de contexte et d'une fonction de calcul de contexte.
